# FASCICULE DE BREVET EUROPEEN

(11) **EP 4 313 923 B1**
(45) Date de publication et mention de la délivrance du brevet: **07.05.2025**
(21) Numéro de dépôt: 22708968.7
(22) Date de dépôt: 09.03.2022
(51) Int. Cl.: C07C 29/80, C07C 29/82, C07C 31/12, C07C 31/10, C07C 31/08, C07C 45/82, B01D 3/00, B01D 3/14, B01D 3/36

(54) **PROCÉDÉ D'EXTRACTION D'ALCOOLS À PARTIR D'UN MÉLANGE INITIAL COMPRENANT DES ALCOOLS EN PHASE AQUEUSE**
VERFAHREN ZUR EXTRAKTION VON ALKOHOLEN AUS EINER ALKOHOLHALTIGEN AUSGANGSMISCHUNG IN WÄSSRIGER PHASE
METHOD FOR EXTRACTING ALCOHOLS FROM AN INITIAL MIXTURE COMPRISING ALCOHOLS IN AQUEOUS PHASE

(30) Priorité: 22.03.2021 FR 2102845
(43) Date de publication de la demande: 07.02.2024
(73) Titulaire: IFP Energies nouvelles, 92500 Rueil-Malmaison (FR)
(72) Inventeur: TOTH, Eszter, 92852 RUEIL-MALMAISON CEDEX (FR); VELLY, Helene, 92852 RUEIL-MALMAISON CEDEX (FR); LOPES FERREIRA, Nicolas, 92852 RUEIL-MALMAISON CEDEX (FR)
(74) Mandataire: IFP Energies nouvelles
(86) Numéro de dépôt international: PCT/EP2022/055967
(87) Numéro de publication internationale: WO 2022/200039

(56) Documents cités:
- FR-A1- 3 053 357

## Description

### Domaine technique

La présente invention concerne un procédé pour extraire, notamment, des alcools d'un mélange comprenant ces alcools en phase aqueuse. On peut l'appliquer à la récupération d'alcools en tant que produits d'un moût obtenu à l'issue de la fermentation d'une solution aqueuse de sucres en C5 et/ou en C6, comme cela est connu des procédés de fermentation dits de type IBE (Isopropanol/Butanol/Ethanol) ou de type IBEA (Isopropanol/Butanol/Ethanol/Acétone) réalisés par des souches « solvantogènes » de type *Clostridium.* On comprend par moût de fermentation un milieu aqueux dans lequel s'est déroulée la fermentation. On comprend par sucres en C5 et/ou en C6 des oses possédant 5 ou 6 carbones. L'invention vise plus spécifiquement à extraire de l'isopropanol et du butanol comme composés majoritaires valorisables.

### Technique antérieure

Afin de répondre aux enjeux de la transition énergétique, de nombreuses recherches sont menées pour développer des procédés dits « verts », permettant d'accéder à des intermédiaires chimiques d'une façon alternative au raffinage du pétrole et/ou à la pétrochimie.

Les alcools issus de procédés fermentaires (par exemple isopropanol et n-butanol) sont parmi les substituts de dérivés pétrochimiques les plus prometteurs. La fermentation ABE (Acétone - Butanol - Ethanol), réalisée par des microorganismes appartenant au genre *Clostridium,* est une des plus anciennes fermentations à avoir été industrialisée, et a été depuis largement étudiée. Plus récemment, la fermentation IBE (Isopropanol - Butanol - Ethanol) produisant un mélange d'isopropanol, butanol et éthanol et réalisée également par des microorganismes appartenant au genre *Clostridium* a fait l'objet d'études assez récentes (Dos Santos Vieira et al Bioresour Technol ; 2019 287:121425. doi:10.1016/j.biortech.2019.121425 Acetone-free biobutanol production: Past and recent advances in the Isopropanol-Butanol-Ethanol (IBE) fermentation).

Concernant le mode de conduite de fermentation employé dans ce type de procédé, a été étudiée la production en mode discontinu (« batch » selon la terminologie anglo-saxonne) pour les fermentations ABE et IBE (voir, par exemple, Jones D. T., Woods D.R., 1986, Acetone-Butanol Fermentation Revisited. Microbiol. Rew., 50 (4), 484-524 ou Tableau 16.6 Lopez-contreras A. et al chapter book 16, Bioalcohol Production: Biochemical Conversion of Lignocellulosic Biomass, 2010).

Ont également été étudiés des procédés opérant en continu, tout d'abord avec des cellules en suspension dans un réacteur homogène. Des améliorations aux procédés continus ont ensuite été proposées en augmentant la rétention de la biomasse microbienne dans le bioréacteur, notamment en utilisant des cellules immobilisées sur un substrat, et/ou en utilisant un recyclage cellulaire avec une rétention au moyen de membranes filtrantes (Vieira et al. 2019 Acetone-free biobutanol production: past and recent advances in the Isopropanol-Butanol-Ethanol (IBE) fermentation Biores. Technol., 287 ; 121425).

Un des verrous rencontrés dans le développement des procédés fermentaires est l'étape de récupération des produits fortement dilués dans le moût fermentaire. C'est un paramètre déterminant au niveau du coût économique de ces types de procédé. Afin de rendre ce procédé de production par voie fermentaire économiquement viable à grande échelle, on cherche, dans un premier temps, à optimiser les performances fermentaires en maximisant le titre final ainsi que la productivité volumique dans le bioréacteur où est réalisée la fermentation. Mais une fois les conditions de la fermentation optimisées, pour une concentration donnée des molécules d'intérêt dans l'eau, il est économiquement important de chercher à améliorer la consommation énergétique liée à la phase d'extraction des molécules d'intérêts du moût de fermentation

Plusieurs techniques existent pour opérer cette extraction, la plus conventionnelle utilise une ou des colonnes de distillation en série, comme FR3053357, même si d'autres techniques, de type strippage du moût par un flux gazeux, ont également été explorées, par exemple dans le brevet WO2018/001628.

L'invention s'intéresse à une extraction par distillation(s). Elle a alors pour but d'améliorer cette extraction. Elle cherche, plus particulièrement, pour une concentration donnée du moût de fermentation, à diminuer la consommation énergétique et/ou les investissements en équipement requis pour cette extraction.

### Résumé de l'invention

L'invention a tout d'abord pour objet un procédé d'extraction d'alcools à partir d'un mélange initial comprenant des alcools, dont au moins de l'isopropanol et du butanol, et optionnellement de l'éthanol et/ou de l'acétone, en phase aqueuse, ledit procédé comportant une série d'opérations de séparation, dont :
- une séparation par distillation opérée par au moins une colonne de distillation dite isopropanol-butanol et visant à séparer ledit mélange initial ou un mélange dérivant dudit mélange initial en tête en un flux d'azéotrope eau-isopropanol et en fond en un flux azéotrope eau-butanol,
- une séparation par distillation visant à séparer le flux d'azéotrope eau-butanol en eau et en butanol, opérée par un système de distillation hétéro-azéotropique comprenant au moins une colonne dite colonne à eau récupérant l'eau et au moins une colonne dite colonne à butanol récupérant le butanol.

Selon l'invention, on opère un transfert de chaleur depuis le flux d'azéotrope eau-isopropanol entrant dans le condenseur de la colonne de distillation isopropanol-butanol vers le flux entrant dans le rebouilleur de la colonne à butanol.

On comprend au sens de l'invention par « un mélange dérivant dudit mélange initial » un mélange qui provient du mélange initial, notamment après au moins un traitement de séparation de type distillation.

Le condenseur de la colonne de distillation isopropanol-butanol et le rebouilleur de la colonne à butanol peuvent avantageusement être intégrés dans un même (et unique) équipement.

L'extraction selon l'invention comporte donc une succession de distillations, chaque colonne de distillation utilisée requérant un apport en chaleur énergivore. Il est connu que les colonnes sont généralement équipées d'un rebouilleur en fond, qui va être chauffé, généralement par de la vapeur d'eau externe, pour monter la température du liquide issu du fond de la colonne à la température voulue et en vaporiser une partie, et générer ainsi une circulation de gaz (« trafic vapeur ») dans la colonne, et d'un condenseur en tête pour faire retomber la température de l'effluent de tête et le faire repasser en phase liquide, et générer ainsi le trafic liquide dans la colonne. Dans le cas d'une succession de colonnes, comme dans le cas présent, le choix de l'invention a été d'intégrer thermiquement deux colonnes de distillation, en sélectionnant celles qui étaient aptes à le faire, à savoir :
- d'une part la colonne à isopropanol-butanol, dont l'effluent de tête comprenant de l'isopropanol pouvait présenter la température suffisante pour transférer de la chaleur, quitte à modifier les conditions opératoires de son fonctionnement pour atteindre cette température,
- d'autre part la colonne à butanol, ayant besoin d'un apport de chaleur suffisant pour monter la charge à une température appropriée pour la distillation, quitte, là encore, à l'ajuster pour permettre ce transfert thermique, sachant qu'un transfert thermique n'est techniquement envisageable que si la différence de températures à atteindre entre l'effluent « chaud » d'une part et la charge « froide » d'autre part est d'au moins 5, et de préférence d'au moins 10°C.

Et ce transfert thermique s'est avéré très efficace pour diminuer la consommation énergétique du procédé dans son ensemble, puisque le rebouilleur de la colonne à butanol est chauffée au moins partiellement, voire totalement, par la chaleur de condensation de la colonne à isopropanol-butanol: on peut diminuer voire supprimer la consommation de vapeur jusque-là nécessaire au chauffage du rebouilleur de la colonne à butanol. Sur le plan des équipements, le gain est également significatif, puisqu'on peut sous-dimensionner, voire supprimer, le moyen de chauffage type four requis pour produire la vapeur d'eau nécessaire au chauffage du rebouilleur.

De façon préférée, la colonne de distillation isopropanol-butanol est équipée en tête d'un condenseur, la colonne à butanol est équipée d'un rebouilleur, et on opère le transfert de chaleur par un échangeur thermique commun aux deux colonnes, intégrant ledit condenseur et ledit rebouilleur, ce qui est particulièrement efficace et économe en moyens d'équipements.

Comme indiqué plus haut, l'intégration thermique proposée par l'invention peut conduire à modifier le fonctionnement, les conditions opératoires (température, pression) de la colonne de distillation isopropanol-butanol, pour que l'effluent eau-isopropanol ait une température suffisante en sortie de colonne pour pourvoir effectuer le transfert.

On peut ainsi modifier, notamment augmenter, la pression de fonctionnement de la colonne. Avantageusement, on peut régler la pression de la colonne de distillation isopropanol-butanol à une valeur d'au moins 3 bars absolus, notamment au moins 4 bars absolus, de préférence d'au plus 10 bars absolus, notamment d'au plus 7 bars absolus, notamment entre 4,5 et 6,5 bars absolus.

On opère de préférence la colonne de distillation isopropanol-butanol et la colonne à butanol à des températures choisies de façon à ce que le flux d'azéotrope eau-isopropanol de tête de la colonne de distillation isopropanol-butanol (le flux « de tête » de cette colonne correspond au flux entrant dans le condenseur de ladite colonne) soit à une température T1 au moins 8°C, notamment au moins 10°C supérieure à la température T2 du flux quittant le fond de la colonne à butanol.

Ainsi, de préférence, on opère la colonne de distillation isopropanol-butanol et la colonne à butanol à des températures choisies de façon à ce que le flux d'azéotrope eau-isopropanol de tête de la colonne de distillation isopropanol-butanol soit à une température T1 d'au moins 120°C, notamment d'au plus 140°C.

On peut régler le fonctionnement de la colonne de distillation isopropanol-butanol pour maintenir un mélange bi-phasique liquide/gaz dans la colonne, notamment en imposant les conditions de température/pression mentionnées plus haut, telle qu'une température du flux eau-isopropanol sortant en tête de colonne d'au moins 120°C et/ou une pression dans la colonne d'au moins 3 bars absolus.

On s'est en effet aperçu que modifier les conditions opératoires de cette colonne (fonctionnant plus habituellement à pression plus basse, notamment à pression atmosphérique, et à température plus basse) permettait du même coup d'obtenir un autre avantage qu'une baisse de coût énergétique : à pression/température plus élevée, la colonne qui tendait à fonctionner en milieu triphasique liquide/liquide/gaz passe en milieu biphasique liquide/gaz seulement, la zone de démixtion disparaît, ce qui permet d'opérer la colonne avec des internes/garnissages habituels/conventionnels et non pas avec des plateaux spécifiques adaptés au fonctionnement des colonnes en triphasique, donc avec un investissement en équipement moindre et un pilotage de la colonne facilité.

Il est à souligner que le surcoût énergétique, le cas échéant, dû à une éventuelle augmentation de pression/température dans la colonne à isopropanol-butanol reste bien inférieur au gain énergétique obtenu pour la colonne à butanol, rendant l'invention très attractive. Il est aussi à souligner que cette augmentation de pression/température dans la colonne à isopropanol-butanol, qui reste maîtrisée, n'est en aucun cas préjudiciable à la qualité, à l'efficacité ou à l'opérabilité de la distillation opérée dans la colonne à isopropanol-butanol en question.

Le mélange à traiter selon l'invention peut aussi contenir un autre alcool, notamment de l'éthanol, généralement en quantité minoritaire par rapport à celle de l'isopropanol et du butanol.

A titre d'exemple, le mélange initial à traiter peut correspondre à un moût fermentaire en phase aqueuse comprenant deux composés majoritaires, à savoir l'isopropanol I et le butanol B, et des composés minoritaires, notamment deux composés minoritaires comme l'acétone A et l'éthanol E, qui présentent les caractéristiques suivantes :
- concentration totale en isopropanol I et butanol B : 8 à 30 g/L
- ratio massiques I/B (produits majoritaires isopropanol/butanol) : 0,25-0,5/0,75-0,5
- concentration totale en produits minoritaires si le moût en contient (par exemple acétone A et éthanol E): 0,1 g/l à 2 g/l.

Dans le cas de figure où le mélange initial comprend aussi de l'éthanol, la séparation par distillation opérée par au moins la colonne de distillation dite isopropanol-butanol vise à séparer ledit mélange ou un mélange dérivant dudit mélange initial en tête en un flux d'azéotrope eau-isopropanol-éthanol et en fond en un flux azéotrope eau-butanol. Le transfert thermique selon l'invention utilise alors la chaleur de condensation du mélange eau-isopropanol-éthanol.

Selon un mode de réalisation préféré, le transfert thermique vers le flux entrant dans le rebouilleur de la colonne à butanol apporte la totalité de la chaleur requise pour le fonctionnement de ladite colonne. Dans ce cas de figure, on peut donc supprimer le four générant la vapeur pour chauffer le rebouilleur de la colonne à butanol.

Selon un autre mode de réalisation, le transfert thermique vers le flux entrant dans le rebouilleur de la colonne à butanol apporte une portion de la chaleur requise au flux entrant dans la colonne à butanol pour le fonctionnement de ladite colonne, le complément de chaleur étant amené par une source de chaleur autre, notamment par une source de vapeur d'eau externe. Dans ce cas de figure, on a donc besoin d'un autre apport de chaleur, soit disponible par ailleurs dans l'installation, soit au moyen d'un four générant de la vapeur, mais alors de plus petite capacité, de plus petite consommation énergétique que sans le transfert thermique selon l'invention.

Selon un autre mode de réalisation, le transfert thermique vers le flux entrant dans la colonne à butanol apporte plus de chaleur que celle requise au flux entrant dans le rebouilleur de la colonne à butanol pour le fonctionnement de ladite colonne, et dans ce cas on ajoute un refroidisseur pour évacuer l'excès de chaleur. On comprend par refroidisseur tout moyen technique connu, par exemple de type condenseur(s).

Un exemple de procédé selon l'invention comprend la série d'opérations suivante de séparation du mélange comprenant des alcools dont au moins de l'isopropanol et du butanol, et éventuellement de l'éthanol, ainsi que de l'acétone, en phase aqueuse :
- (a) une séparation dudit mélange, par distillation opérée dans une colonne à bière visant à éliminer au moins une portion de l'eau de la phase aqueuse pour obtenir un mélange concentré,
- (b) une séparation du mélange concentré à l'étape (a), par distillation opérée dans une colonne dite à acétone visant à séparer l'acétone dudit mélange concentré pour obtenir un mélange concentré appauvri en acétone,
- (c) une séparation par distillation opérée par au moins une colonne de distillation dite isopropanol-butanol et visant à séparer le mélange concentré appauvri en acétone obtenu à l'étape (b) en tête en un flux d'azéotrope eau-isopropanol ou eau-isopropanol-éthanol et en fond en un flux azéotrope eau-butanol,
- (d) une séparation par distillation visant à séparer le flux d'azéotrope eau-butanol obtenu à l'étape (c) en eau et en butanol, opérée par un système de distillation hétéro-azéotropique comprenant au moins une colonne dite colonne à eau récupérant l'eau et au moins une colonne dite colonne à butanol récupérant le butanol.

Avantageusement, le mélange initial comprenant des alcools, dont au moins de l'isopropanol et du butanol, et optionnellement de l'éthanol et/ou de l'acétone, en phase aqueuse est un moût obtenu par fermentation de jus sucrés, notamment dérivés de biomasse lignocellulosique, sous l'action de microorganismes, notamment issus d'une souche solvantogène, de préférence choisis parmi un au moins des microorganismes suivants : des bactéries, notamment du genre *Clostridium* (comme C. *tyrobutyricum* ou C. *cellulovorans*), *Escherichia coli,* des levures, notamment de type *Saccharomyces cerevisae.* Naturellement, ces microorganismes peuvent être les microorganismes natifs, ou en dériver par modification génétique selon des techniques connues.

Ce moût présente en effet la particularité d'être fortement dilué à l'eau, ce qui nécessite ces diverses séparations, et notamment une étape de concentration telle que l'étape (a) mentionnée plus haut.

Le mélange initial traité selon l'invention peut ainsi présenter une concentration de composés organiques de 2 à 40 grammes/litre, notamment de 5 à 35 g/litres ou de 8 à 30 grammes/litre, typiques des concentrations rencontrées dans les moûts de fermentation. On comprend par composés organiques les molécules d'intérêt que l'invention cherche à séparer et à valoriser, notamment de la famille des alcools et/ ou des solvants.

Selon un mode de réalisation de l'invention, le mélange initial (1) ne provient que d'une source, que d'un procédé de production à partir d'une seule charge ; par exemple il s'agit d'un moût de fermentation obtenu à partir d'une seule fermentation avec un seul type de microorganisme, le moût comprenant, de par le procédé choisi, directement le mélange de composés organiques, d'alcools que l'invention cherche à séparer.

Selon un autre mode de réalisation, le mélange initial selon l'invention peut associer plusieurs mélanges de compositions différentes et comprenant chacun un ou des alcools en phase aqueuse. Il peut s'agir de mélanger, notamment, plusieurs moût obtenus par fermentation de jus sucrés avec des microorganismes différents, chaque moût ayant alors sa composition propre. Le mélange de ces différents flux, de ces différents moûts par exemple, peut soit être réalisé préalablement à tout traitement de séparation propre à l'invention, ou dans une section dédiée au prémélange, soit être réalisé lors d'une étape de séparation dans le déroulement du procédé selon l'invention : les différents flux/moûts peuvent par exemple être injectés conjointement dans un dispositif de séparation, par exemple dans la première colonne de distillation de l'installation selon l'invention (la colonne à bière par exemple, comme décrit plus loin), le mélange se faisant alors directement dans le dispositif en question sans prémélange.

On peut ainsi prévoir : - d'une part, de produire un premier moût sous forme d'un premier mélange comprenant de l'isopropanol par fermentation, et - d'autre part, de produire un deuxième moût sous forme d'un deuxième mélange comprenant du butanol par une autre fermentation, et de réunir les deux moûts pour qu'ils soient traités conjointement selon l'invention, (en étant mélangés en amont de l'installation ou dans l'installation mettant en œuvre l'invention, le mélange étant de facto réalisé lors de leur traitement par la colonne à isopropanol-butanol). Chacun des deux moûts peut comprendre des impuretés différentes, ou d'autres molécules d'intérêt (éthanol...).

On vient ainsi mutualiser la séparation de plusieurs moûts différents par une seule et même installation, un seul et même procédé.

Ainsi, pour produire un premier moût comprenant surtout de l'isopropanol, on peut se reporter à l'article « Employing Escherichia coli for C2-C6 Bioalcohol Production », L.Liang et al ., Front.Bioeng.Biotechnol., 03.07.2020).

Et pour produire un deuxième moût comprenant surtout du butanol, on peut se reporter à l'article « Genetic engineering of non-native hosts for 1- butanol production and its challenges : a review », S. Nawab et al., Microb Cell Fact , 27.03.2020.

L'invention a également pour objet toute installation d'extraction d'alcools mettant en œuvre le procédé décrit précédemment.

L'invention a également pour objet une installation d'extraction d'alcools à partir d'un mélange initial comprenant des alcools, dont au moins de l'isopropanol et du butanol, et éventuellement de l'éthanol et/ou de l'acétone, en phase aqueuse, ladite installation comportant une série de sections de séparation, dont :
- une section de séparation par distillation opérée par au moins une colonne de distillation dite isopropanol-butanol équipée d'un condenseur et visant à séparer en tête ledit mélange initial ou un mélange dérivant dudit mélange initial en un flux d'azéotrope eau-isopropanol ou eau-isopropanol-éthanol et en fond en un flux azéotrope eau-butanol,
- une section de séparation par distillation visant à séparer le flux d'azéotrope eau-butanol en eau et en butanol, opérée par un système de distillation hétéro-azéotropique comprenant au moins une colonne dite colonne à eau récupérant l'eau et au moins une colonne dite colonne à butanol équipée d'un rebouilleur et récupérant le butanol,
telle qu'elle prévoit un transfert thermique depuis le flux d'azéotrope eau-isopropanol ou eau-isopropanol-éthanol de tête de la colonne de distillation isopropanol-butanol entrant dans le condenseur vers le flux entrant dans le rebouilleur de la colonne à butanol.

De préférence, l'installation comprend un échangeur thermique commun à la colonne de distillation isopropanol-butanol et à la colonne à butanol et assurant un transfert thermique depuis le flux d'azéotrope eau-isopropanol ou eau-isopropanol-éthanol entrant dans le condenseur de la colonne de distillation isopropanol-butanol vers le flux entrant dans le rebouilleur de la colonne à butanol, cet échangeur commun intégrant ledit condenseur et ledit rebouilleur.

L'installation peut comprendre la série suivante de sections de séparation du mélange initial comprenant des alcools dont au moins de l'isopropanol et du butanol, et éventuellement de l'éthanol, ainsi que de l'acétone en phase aqueuse :
- (a) une section de séparation dudit mélange, par distillation comprenant au moins une colonne à bière visant à éliminer une portion au moins de l'eau de la phase aqueuse pour obtenir un mélange concentré,
- (b) une section de séparation du mélange concentré à l'étape a), par distillation comprenant au moins une colonne dite à acétone visant à séparer l'acétone dudit mélange concentré pour obtenir un mélange concentré appauvri en acétone,
- (c) une section de séparation par distillation comprenant au moins une colonne de distillation dite isopropanol-butanol et visant à séparer le mélange concentré appauvri en acétone obtenu à l'étape (b) en tête en un flux d'azéotrope eau-isopropanol ou eau-isopropanol-éthanol et en fond en un flux azéotrope eau-butanol,
- (d) une section de séparation par distillation visant à séparer le flux d'azéotrope eau-butanol obtenu à l'étape (c) en eau et en butanol, comprenant au moins un système de distillation hétéro-azéotropique comprenant au moins une colonne dite colonne à eau pour récupérer l'eau et au moins une colonne dite colonne à butanol pour récupérer le butanol.

L'installation selon l'invention peut aussi comprendre un condenseur supplémentaire en tête de la colonne à isopropanol-butanol pour évacuer l'excès de chaleur du flux d'azéotrope eau-isopropanol ou eau-isopropanol-éthanol de tête, dans le cas où la quantité de chaleur disponible dans le flux d'azéotrope dépasse la quantité de chaleur nécessaire au flux entrant dans le rebouilleur de la colonne à butanol.

L'invention sera décrite ci-après de façon plus détaillée, à l'aide d'exemples non limitatifs et des figures suivantes :

### Liste des figures

Figure 1

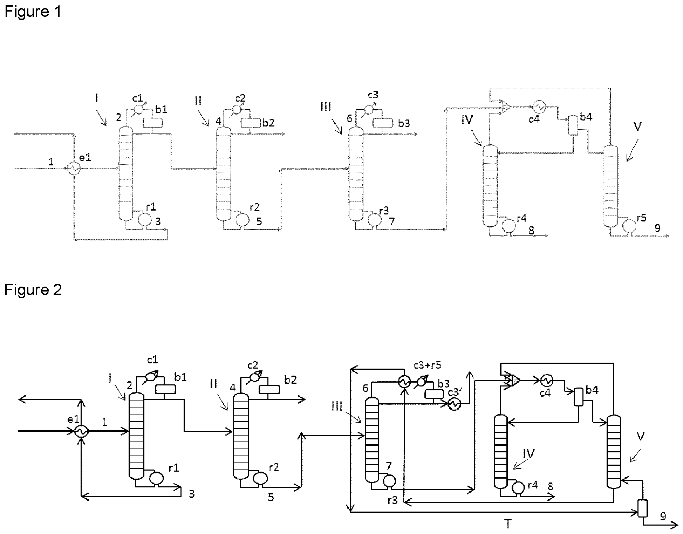

   La figure 1 représente un exemple d'installation de séparation de molécules d'intérêt en solution aqueuse de l'état de la technique.
Figure 2
   La figure 2 représente l'installation de la figure 1 modifiée selon un mode de réalisation non limitatif de l'invention.

Les références identiques d'une figure à l'autre représentent les mêmes flux, dispositifs et échanges thermiques.

Ces deux figures sont extrêmement schématiques : ce sont des schémas de principe, qui ne sont pas à l'échelle. Les installations sont représentées de façon simplifiée pour en faciliter la lecture, notamment pour bien saisir les dispositifs/flux exploités par l'invention, sans représenter tous les dispositifs, de type fours, échangeurs, refroidisseurs/compresseurs, rebouilleurs de colonne, condenseurs de colonne etc... effectivement prévus dans une installation industrielle de ce type et connus de l'homme du métier.

Dans toutes les figures :
- les références numérales désignent des flux de fluide, les références sous forme de chiffres romains désignent les colonnes de distillation, les références avec des lettres les équipements,
- par soucis de clarté, les « sections de séparation » sont représentées avec une unique colonne de type colonne de distillation. Mais il est clair que les sections de séparation peuvent contenir/contiennent une pluralité de colonnes, montées en série et/ou en parallèle, et qu'elles peuvent contenir d'autres dispositifs de séparation pour compléter le rôle d'une au moins des colonnes de distillation, par exemple un ou des séparateurs liquide/gaz du type ballons etc....

### Description des modes de réalisation

L'objectif de l'invention est, dans l'exemple suivant non limitatif, de récupérer les produits majoritaires, à savoir l'isopropanol d'une part et le n-butanol d'autre part, d'une solution aqueuse, qui contient également deux autres composants minoritaires, à savoir de l'acétone, considérée ici comme une impureté à éliminer, et de l'éthanol, que l'on choisit ici de conserver avec l'isopropanol.

La composition du moût initial (entrant dans la colonne à bière), comme indiqué plus haut, est la suivante :
- concentration totale en isopropanol I et butanol B : 8 à 30 g/L
- ratio massique I/B (produits majoritaires isopropanol/butanol) : entre 0,25-0,5/ 0,75-0,5
- concentration totale en produits minoritaires acétone a et éthanol e: 0,1 à 2 g/l.

Le moût de fermentation dont part l'invention, à titre d'exemple non limitatif, a la composition et les caractéristiques suivantes :
- concentration en isopropanol I, butanol B, éthanol e, et acétone a dans l'eau : 19,6 g/l
- ratio en poids I/B/e+a = 40,8/56,1/3,1
- concentration en acétone a environ double de celle de l'éthanol e

Cette solution aqueuse est un moût de fermentation issu d'un jus composé de sucres C5 et/ou C6 et fermenté sous l'action de microorganismes dérivés d'une souche de genre Clostridium, de façon connue, par exemple selon les procédés « batch » ou continus évoqués en préambule. Pour plus de détails sur le processus de fermentation à proprement dit, on pourra se reporter à la publication scientifique citée pour le procédé « batch ».

La composition du flux entrant dans la colonne à isopropanol-butanol est notamment la suivante :
- eau : 35 à 45 %poids
- le reste étant constitué d'IBea
- le ratio r massique I/B (produits majoritaires isopropanol/butanol) : 0,25-0,5 / 0,75-0,5
- concentration en acétone a : 0,1 à 1 g/l
- concentration en éthanol e : 2 à 10 g/l

L'isopropanol obtenu peut avoir deux utilisations :
- il peut être transformé en propylène. En effet, le produit isopropanol hydraté obtenu (avec l'éthanol comme produit minoritaire) avec le procédé et l'installation selon l'invention est une matière première adaptée à une conversion en propylène, car conduisant à la production d'une coupe isopropanol (et un peu d'éthanol) hydratée, car l'isopropanol présente un azéotrope avec l'eau qu'il est impossible de séparer complètement uniquement par distillation.
- il peut aussi être utilisé comme solvant, après l'élimination de l'eau. Il faut alors envisager un procédé de déshydratation supplémentaire pour éliminer l'eau de l'azéotrope isopropanol/eau. On peut citer la distillation avec tierce corps (benzène, cyclohexane...), la « pressure swing distillation », (acronyme PSD), qui est un terme anglo-saxon désignant une distillation à deux pressions différentes, la « Temperature Swing Adsorption » (acronyme TSA) qui est un terme anglo-saxon pour désigner une adsorption modulée en température ou la « Pressure Swing Adsorption » (acronyme PSA) qui est un terme anglo-saxon désignant une adsorption modulée en pression, ou la pervaporation.

Le butanol obtenu (n-butanol ici), quant à lui, est produit quasi-pur dans le procédé et l'installation selon l'invention. On comprend par « quasi pur » une teneur en butanol d'au moins 98 % en poids, notamment d'au moins 98,5 ou 99 % dans la phase liquide considérée (ou une teneur en impuretés d'au plus 2 %, notamment d'au plus 1,5 ou 2% en poids).

On pourra, dans le présent texte, désigner le mélange d'isopropanol, butanol, éthanol, et acétone dans l'eau sous le terme de mélange « IBea » ou simplement sous le terme « IBea » par soucis de concision.

### Exemples

### Exemple 1 (comparatif)

Cet exemple correspond à la mise en œuvre de l'installation selon la figure 1.

L'installation de séparation représentée à la figure 1 va être décrite d'«amont» en «aval», en comprenant ces termes selon la direction d'écoulement global du moût de fermentation et des produits extraits à travers l'installation.

Le moût 1 arrive depuis la section de fermentation (non représentée) avec une concentration en mélange IBea d'environ 8 à 30 g/L et une température comprise entre 34 et 37°C.

La première colonne I, appelée usuellement colonne à bière, pré-concentre le moût 1 jusqu'à environ 60% poids d'IBea, sortant en tête de colonne sous forme d'un flux 2 de mélange IBea concentré, et élimine en fond de colonne environ 97 à 99 % de l'eau entrante. Cette eau 3 de fond de colonne I contient de 50 à 1000 ppm en poids d'IBea et est appelée « Vinasses ». Cette colonne I fonctionne, de façon conventionnelle, sensiblement à la pression atmosphérique, elle peut aussi être opérée au maximum à 3 bars absolus. Elle a entre 10 et 20 plateaux théoriques. Elle représente environ 70 à 90 % de la consommation de vapeur du procédé complet de séparation (on comprend par ce terme le procédé mis en œuvre dans une installation telle que représentée à la figure 1). La colonne I opère, en fonction de sa pression, dans une gamme de températures d'environ 100 à 140 °C (hors condenseur). On peut placer un échangeur charge/effluent sur cette colonne I afin de préchauffer la charge (le moût 1).

Celle-ci, initialement à 34-37 °C, rentre dans la colonne I, après être passée par l'échangeur charge /effluent e1, à une température comprise entre 70 et 85°C.

La deuxième colonne II, appelée colonne à Acétone a pour vocation d'éliminer l'acétone qui est ici considérée comme une impureté. Elle fonctionne de façon conventionnelle, sensiblement à la pression atmosphérique, peut aussi être opérée au maximum à 3 bars absolus. Elle comprend entre 30 et 50 plateaux théoriques. Elle représente environ 2 à 6 % de la consommation de vapeur du procédé global de séparation. Le flux 2 entre dans la colonne II. En sortent, en tête, le flux d'acétone 4, et en fond, le flux appauvri en acétone 5.

La troisième colonne III, appelée colonne à isopropanol-butanol, permet d'obtenir l'azéotrope isopropanol/eau en tête, c'est le flux 6, qui comprend aussi le peu d'éthanol entrant avec le moût comme composé minoritaire. La composition de cet azéotrope est entre 11 et 15 % poids en eau. Au fond de la colonne on obtient un flux d'une composition proche de celle de l'azéotrope eau/n-butanol soit une composition d'environ 50%/50% (poids), c'est le flux 7. Cette colonne III fonctionne ici à pression atmosphérique, et la colonne a une pression de tête de 1,5 bars absolus. Elle comporte entre 30 et 70 plateaux théoriques, dans cet exemple elle en comprend 50. Dans ces conditions de pression, on observe une zone triphasique vapeur/liquide/liquide entre les plateaux 27 et 50, en commençant la numérotation des plateaux au niveau du condenseur c3, avec des plateaux spécifiques au mode de fonctionnement triphasique de la colonne. Cette colonne III représente environ 7 à 15 % de de la consommation de vapeur du procédé complet de séparation.

Les deux dernières colonnes IV et V sont couplées : il s'agit d'un système de distillation hétéro-azéotropique, connue dans son principe, appliquée au binaire eau/n-butanol. Au fond de la colonne IV à eau, on obtient l'eau (flux 8) contenu dans l'hétéro-azéotrope et au fond de la colonne V à butanol, on obtient le n-butanol (flux 9) avec une pureté pouvant aller de 98 % à 99,5 % poids. Les deux colonnes fonctionnent sensiblement à la pression atmosphérique, pouvant aller jusqu'à 2 bars absolus. Les deux colonnes IV et V ont entre 7 et 15 plateaux théoriques. La colonne IV à eau représente 1 à 3 % de la consommation de vapeur du procédé complet de séparation, et la colonne V à butanol représente 4 à 8% du procédé complet de séparation.

Toutes les colonnes I à V sont équipées, de façon connue pour des colonnes de distillation,
- d'un condenseur c1, c2, c3, c4 en tête de colonne, le dernier condenseur c4 étant commun aux colonnes IV et V,
- et d'un rebouilleur r1, r2, r3, r4, r5 en bas de colonne pour chauffer le flux de fond de colonne. De façon connue, ces rebouilleurs peuvent être choisis parmi les rebouilleurs suivants : les rebouilleurs à thermosiphon vertical, les rebouilleurs dits de type « kettle » (« bouilloire » en français), les rebouilleurs par four ou encore les rebouilleurs dits « once-through » ou « traversant» en français
- et éventuellement d'un ballon de reflux b1, b2, b3, b4 en aval des condenseurs c1 à c4.

La consommation de vapeur du procédé complet de séparation tel que décrit varie entre 12 et 50 MJ vapeur/kg d'IBea. Elle dépend de la concentration en IBea de la charge initiale 1 (c'est-à-dire du moût récupéré après fermentation), ainsi que des divers choix de conception de l'installation (conditions opératoires de fonctionnement des colonnes, conception des colonnes du type nombre de plateaux, position des alimentations etc...).

En termes de consommation énergétique des colonnes à isopropanol III et à butanol V:
- le condenseur c3 de la colonne III à isopropanol doit évacuer 1,43 MJ/kg IBea de chaleur disponible à une température de l'ordre de 80°C,
- le rebouilleur r3 de cette même colonne III consomme 1,48 MJ/kg IBea de chaleur sous forme de vapeur de rebouillage,
- la colonne V à butanol consomme 0,9 MJ/kg IBea de chaleur sous forme de vapeur de rebouillage (rebouilleur r5). Cette chaleur est requise à un niveau thermique d'environ 120°C à la pression de fonctionnement considérée.

### Exemple 2 (selon l'invention)

Cet exemple correspond à la mise en oeuvre de l'installation selon la figure 2, qui modifie et améliore l'installation de la figure 1. Ne seront détaillées/précisées que les différences avec le schéma d'installation de la figure 1, toutes choses égales par ailleurs.

Selon cet exemple 2, un transfert thermique est ajouté depuis la colonne III à isopropanol-butanol vers la colonne V à butanol. Pour ce faire, on augmente la pression de la colonne III : au lieu de l'opérer à pression atmosphérique, on choisit de l'opérer en pression, entre 4 et 7 bars absolus, et plus précisément ici vers 5,5 bars absolus (mesurée en tête de colonne). Cette augmentation de pression conduit à une augmentation des températures de fonctionnement de la colonne III. Ainsi, la température de tête de colonne augmente jusqu'à au moins 120°C, notamment jusqu'à 125°C à 130°C, alors qu'avec le fonctionnement standard proche de la pression atmosphérique selon l'exemple 1, elle n'est que de l'ordre de 80°C. Or, en tête de colonne, on doit évacuer des calories, et avec cette augmentation de température, il s'avère que la quantité de calories à évacuer de l'effluent de tête dans le condenseur c3 de la colonne III est proche, voire un peu supérieure, à la quantité de calories à fournir au niveau du rebouilleur r5 de la colonne V à butanol, qui fonctionne vers 120°C à pression atmosphérique.

En augmentant la température de tête de la colonne III, on peut transférer la chaleur évacuée au niveau du condenseur c3 de cette colonne III vers le rebouilleur r5 de la colonne butanol, qui fonctionne vers environ 120°C à la pression proche de l'atmosphérique, via une nouvelle connexion thermique T. On n'a alors besoin que d'un échangeur commun aux deux colonnes constituant à la fois le condenseur c3 de la colonne III et le rebouilleur r5 de la colonne V, que l'on peut regrouper en un même équipement c3+r5. On remplace ainsi deux équipements (condenseur de la colonne III à isopropanol-butanol et le rebouilleur de la colonne V à butanol) par un seul et même échangeur de chaleur Et on peut supprimer le moyen de production de vapeur associé au rebouilleur r5 de la colonne 5.

Dans le cas où la quantité de chaleur à évacuer au niveau de la colonne III n'est pas rigoureusement égale à la quantité de chaleur à fournir à la colonne V, on a plusieurs options :
- Si elle est supérieure, on ajoute un condenseur supplémentaire c3' (par exemple un refroidisseur du type de ceux désignés sous le terme anglo-saxon de « trim cooler ») en tête de colonne III, qui sera plus petit que le condenseur de tête de colonne c3 initial , car il doit évacuer moins de chaleur que le condenseur c3 de l'exemple 1. L'exemple 2 et la figure 2 représente ce cas de figure. L'intégralité du flux de tête 6 de III entre dans l'échangeur commun c3+r5, intégrant le condenseur c3 de la colonne III et le rebouilleur r5 de la colonne V. L'effluent chaud sortant de cet échangeur commun entre dans le condenseur supplémentaire c3'. En sortie de ce condenseur c3', le flux est divisé en 2 : une portion repart en reflux dans la colonne III, la portion restante, le distillat, sort du procédé, c'est un produit.
- Si elle est inférieure, on doit prévoir un appoint de vapeur au niveau du rebouilleur r5 de la colonne V, donc utiliser un moyen de chauffe (non représenté à la figure) pour produire de la vapeur, mais cet appoint reste significativement inférieur à la quantité de vapeur nécessaire sans ce transfert thermique entre les deux colonnes, on pourra donc utiliser un moyen de chauffe (type four) bien plus petit, de capacité de chauffe bien moindre que dans le cas de l'exemple 1.

De façon surprenante, il s'est avéré qu'augmenter la pression de la colonne III apportait un autre avantage : à pression atmosphérique, il existe une zone de démixtion liquide / liquide dans cette colonne, due à la présence du ternaire Isopropanol/ n-Butanol/Eau. Or, ce type de zone triphasique liquide/liquide/vapeur requiert généralement la mise en place d'internes de colonnes spécifiques, plus coûteux et plus complexes de mise en œuvre que des plateaux de distillation conventionnels. Avec la pression augmentée selon l'invention, la zone de démixtion triphasique disparait dans la colonne, et donc on peut opérer la colonne avec des internes conventionnels de type plateaux à clapets ou perforés, des garnissages vrac ou structurés ce qui permet de diminuer encore les investissements d'installation.

En termes de consommation énergétique des colonnes III à isopropanol-butanol et V à butanol :
- le condenseur c3 de cette troisième colonne III doit évacuer 1,44 MJ/kg IBea de chaleur, disponible à une température de 129,5°C, cette hausse de température par rapport à l'exemple 1 découlant de la hausse de la pression de fonctionnement de la colonne.

Or la cinquième colonne V à butanol requiert une chaleur de rebouillage de 0,9 MJ/kg IBea sous forme de vapeur, à un niveau thermique de 119,5°C. Le transfert thermique entre le condenseur de la colonne III et le bouilleur de la colonne V est devient donc possible. Ici, il faudra évacuer 1,44 - 0,9 = 0,54 MJ/kg IBea au niveau du condenseur c3 de la colonne III à isopropanol-butanol (au lieu des 1,43 MJ/kg IBea selon l'exemple 1) : la chaleur à évacuer est moindre que dans le cas de l'exemple 1 et l'équipement sera plus petit. Enfin, la chaleur à fournir au rebouilleur r3 de la colonne III isopropanol est de 1,71 MJ/kg au lieu des 1,48 MJ/kg, soit une hausse de 0,23 MJ/kg (due à la hausse de pression).

Le tableau 1 ci-dessous récapitule les données énergétiques des colonnes III et V selon l'exemple 1 et l'exemple 2, et détaille le calcul des gains énergétiques qui en découlent:

**Tableau 1**

| | Exemple 1 (comparatif) | Exemple 2 (Invention) | Gain G1,G2 |
|---|---|---|---|
| **Colonne III à Isopropanol/ Butanol** | | | |
| Pression de tête (bars abs.) | 1,5 | 5,5 | |
| Condenseur c3 (MJ/kg IBea) | 1,43 | 1,44 | |
| Rebouilleur r3 (MJ/kg IBea) | 1,48 | 1,71 | |

| **Colonne V à Butanol** | | | |
|---|---|---|---|
| Pression de tête (bars abs) | 1,15 | 1,15 | |
| Rebouilleur r5 (MJ/kg IBea) | 0,9 | 0,9 | |
| Chaleur à évacuer (MJ/kg IBea) | 1,43 | 1,44 - 0,9 = 0,54 | G2 = 1,43 - 0,54 = 0,89 |
| Chaleur à fournir (MJ/ kg IBea) | 1,48 + 0,9 = 2,38 | 1,71 + 0,9 - 0,9 = 1,71 | G1 = 2,38 -1,71 = 0,67 |

En comparant ces données sur le fonctionnement énergétique des colonnes III et V, on vérifie qu'avec l'invention, on gagne à la fois :
- en termes d'énergie pour évacuer la chaleur de la colonne III, avec un gain G2 sur la chaleur à évacuer de 0,89 MJ/kg IBea, ce qui correspond à une diminution de la consommation énergétique de refroidissement de (0,89/1,43 x 100 =) 62 % ;
- et en termes d'énergie à fournir à la colonne V, avec un gain G1 sur la chaleur à fournir de 0,67 MJ/kg IBea, ce qui correspond à une diminution de la consommation énergétique de chauffe de (0,67/2,38 x 100 =) 28%.

On voit donc que globalement le transfert thermique entre les deux colonnes de distillation selon l'invention aboutit à des gains énergétiques considérables par rapport à une utilisation conventionnelle de ces deux colonnes, aussi bien en refroidissement (diminution spectaculaire de plus de la moitié de la consommation), qu'en chauffage (diminution significative d'au moins 20%), sans entraîner de surcoût notable en termes d'équipement. Le condenseur c3 et le rebouilleur r5 ayant été intégrés dans un seul équipement et la chaleur à évacuer au niveau du condenseur supplémentaire c3' (le « trim-cooler ») étant 62% plus faible, ce condenseur supplémentaire c3' de l'exemple 2 selon l'invention est/peut être nettement plus petit que le condenseur c3 de l'exemple 1. A noter que l'ajout de ce condenseur supplémentaire reste optionnel, selon les cas de figure rencontrés, comme indiqué plus haut.

## Revendications

1. Procédé d'extraction d'alcools à partir d'un mélange initial (1) comprenant des alcools, dont au moins de l'isopropanol et du butanol, et optionnellement de l'éthanol et/ou de l'acétone, en phase aqueuse, ledit procédé comportant une série d'opérations de séparation, dont :
- une séparation par distillation opérée par au moins une colonne de distillation dite à isopropanol-butanol (III) équipée en tête d'un condenseur (c3) et visant à séparer ledit mélange initial ou un mélange dérivant dudit mélange initial en tête en un flux (6) d'azéotrope eau-isopropanol et en fond en un flux (7) azéotrope eau-butanol,
- une séparation par distillation visant à séparer le flux d'azéotrope eau-butanol en eau et en butanol, opérée par un système de distillation hétéro-azéotropique comprenant au moins une colonne dite colonne à eau (IV) récupérant l'eau et au moins une colonne dite colonne à butanol (V) équipée d'un rebouilleur (r5) et récupérant le butanol (9),
**caractérisé en ce qu'**on opère un transfert de chaleur depuis le flux d'azéotrope eau-isopropanol (6) de tête entrant dans le condenseur de la colonne de distillation isopropanol-butanol (III) vers le flux entrant dans le rebouilleur de la colonne à butanol (V).

2. Procédé selon la revendication précédente, **caractérisé en ce qu'**on opère le transfert de chaleur par un échangeur thermique commun (c3+r5) aux deux colonnes (III,V), intégrant ledit condenseur (c3) et ledit rebouilleur (r5).

3. Procédé selon l'une des revendications précédentes, **caractérisé en ce qu'**on règle le fonctionnement de la colonne de distillation à isopropanol-butanol (III) pour maintenir un mélange bi-phasique liquide/gaz dans la colonne, notamment en imposant une température d'au moins 120°C et/ou une pression d'au moins 3 bars absolus.

4. Procédé selon l'une des revendications précédentes, **caractérisé en ce qu'**on règle la pression de la colonne de distillation à isopropanol-butanol (III) à une valeur d'au moins 3 bars absolus, notamment au moins 4 bars absolus, de préférence d'au plus 10 bars absolus, notamment d'au plus 7 bars absolus, notamment entre 4,5 et 6,5 bars absolus.

5. Procédé selon l'une des revendications précédentes, **caractérisé en ce qu'**on opère la colonne de distillation à isopropanol-butanol (III) et la colonne à butanol (IV) à des températures choisies de façon à ce que le flux (6) d'azéotrope eau-isopropanol de tête de la colonne de distillation à isopropanol-butanol (III) soit à une température T1 au moins 8°C, notamment au moins 10°C supérieure à la température T2 du flux quittant le fond de la colonne à butanol (V).

6. Procédé selon l'une des revendications précédentes, **caractérisé en ce qu'**on opère la colonne de distillation à isopropanol-butanol (III) et la colonne à butanol (V) à des températures choisies de façon à ce que le flux (6) d'azéotrope eau-isopropanol de tête de la colonne de distillation isopropanol-butanol (III) soit à une température T1 d'au moins 120°C, notamment d'au plus 140°C.

7. Procédé selon l'une des revendications précédentes, **caractérisé en ce que** le mélange initial (1) comprend aussi de l'éthanol, et **en ce que** la séparation par distillation qui est opérée par au moins la colonne de distillation à isopropanol-butanol (III) vise à séparer ledit mélange ou un mélange dérivant dudit mélange initial en tête en un flux d'azéotrope eau-isopropanol-éthanol (6) et en fond en un flux (7) azéotrope eau-butanol.

8. Procédé selon l'une des revendications précédentes, **caractérisé en ce que** le transfert thermique vers le flux entrant dans la colonne à butanol (V) apporte la totalité de la chaleur requise au flux entrant dans le rebouilleur (r5) de la colonne à butanol (V) pour le fonctionnement de ladite colonne.

9. Procédé selon l'une des revendications 1 à 7, **caractérisé en ce que** le transfert thermique vers le flux entrant dans la colonne à butanol (V) apporte une portion de la chaleur requise au flux entrant dans la colonne à butanol (V) pour le fonctionnement de ladite colonne, le complément de chaleur étant amené par une source de chaleur autre, notamment par une source de vapeur d'eau externe.

10. Procédé selon l'une des revendications 1 à 7, **caractérisé en ce que** le transfert thermique vers le flux entrant dans la colonne à butanol (V) apporte plus de chaleur que celle requise au flux entrant dans le rebouilleur (r5) de la colonne à butanol (V) pour le fonctionnement de ladite colonne, et **en ce qu'**on ajoute un refroidisseur (c3') pour évacuer l'excès de chaleur.

11. Procédé selon l'une des revendications précédentes, **caractérisé en ce qu'**il comprend la série d'opérations suivante de séparation du mélange initial (1) comprenant des alcools dont au moins de l'isopropanol et du butanol, et éventuellement de l'éthanol, ainsi que de l'acétone, en phase aqueuse :
- (a) une séparation dudit mélange, par distillation opérée dans une colonne à bière (I) visant à éliminer au moins une portion de l'eau de la phase aqueuse pour obtenir un mélange concentré (2),
- (b) une séparation du mélange concentré issu de l'étape (a), par distillation opérée dans une colonne dite à acétone (II) visant à séparer au moins en partie l'acétone dudit mélange concentré (2) pour obtenir un mélange concentré appauvri en acétone (5),
- (c) la séparation par distillation opérée par au moins une colonne de distillation dite à isopropanol-butanol (III) et visant à séparer le mélange concentré appauvri en acétone obtenu à l'étape (b) en tête en un flux (6) d'azéotrope eau-isopropanol ou eau-isopropanol-éthanol et en fond en un flux (7) azéotrope eau-butanol,
- (d) la séparation par distillation visant à séparer le flux d'azéotrope eau-butanol obtenu à l'étape (c) en eau et en butanol, opérée par un système de distillation hétéro-azéotropique comprenant au moins une colonne dite colonne à eau (IV) récupérant l'eau et au moins une colonne dite colonne à butanol (V) récupérant le butanol.

12. Procédé selon l'une des revendications précédentes, **caractérisé en ce que** le mélange initial (1) comprenant des alcools, dont au moins de l'isopropanol et du butanol, et optionnellement de l'éthanol et/ou de l'acétone, en phase aqueuse est un moût obtenu par fermentation de jus sucrés, notamment dérivés de biomasse lignocellulosique, sous l'action de microorganismes, notamment issus d'une souche solvantogène, de préférence choisis parmi un au moins des microorganismes suivants : des bactéries, notamment du genre *Clostridium, Escherichia coli,* des levures, notamment de type *Saccharomyces cerevisae.*

13. Procédé selon l'une des revendications précédentes, **caractérisé en ce que** le mélange initial (1) associe plusieurs mélanges de compositions différentes et comprenant chacun un ou des alcools en phase aqueuse, notamment plusieurs moût différents obtenus par fermentation de jus sucrés.

14. Installation d'extraction d'alcools à partir d'un mélange initial (1) comprenant des alcools, dont au moins de l'isopropanol et du butanol, et éventuellement de l'éthanol et/ou de l'acétone, en phase aqueuse, ladite installation comportant une série de sections de séparation, dont :
- une section de séparation par distillation opérée par au moins une colonne de distillation (III) dite à isopropanol-butanol équipée en tête d'un condenseur (c3)et visant à séparer ledit mélange initial ou un mélange dérivant dudit mélange initial en tête en un flux (6) d'azéotrope eau-isopropanol ou eau-isopropanol-éthanol et en fond en un flux (7) azéotrope eau-butanol,-une section de séparation par distillation visant à séparer le flux (7) d'azéotrope eau-butanol en eau et en butanol, opérée par un système de distillation hétéro-azéotropique comprenant au moins une colonne dite colonne à eau récupérant l'eau (8) et au moins une colonne dite colonne à butanol équipée d'un rebouilleur (r5) et récupérant le butanol (9), **caractérisé en ce que** l'installation comprend un échangeur thermique commun à la colonne de distillation isopropanol-butanol (III) et à la colonne à butanol (V) et assurant un transfert thermique depuis le flux (6) d'azéotrope eau-isopropanol ou eau-isopropanol-éthanol de tête de la colonne de distillation à isopropanol-butanol (III) entrant dans le condenseur (c3) vers le flux entrant dans le rebouilleur (r5) de la colonne à butanol (V), ledit échangeur thermique commun aux deux colonnes de distillation à isopropanol-butanol (III) et à butanol (V) intégrant ledit condenseur (c3) et ledit rebouilleur (r5), et optionnellement un condenseur supplémentaire (c3') en tête de la colonne à isopropanol-butanol (III) pour évacuer l'excès de chaleur du flux (6) d'azéotrope eau-isopropanol ou eau-isopropanol-éthanol de tête.

15. Installation selon la revendication 14, **caractérisée en ce qu'**elle comprend la série suivante de sections de séparation du mélange initial comprenant des alcools dont au moins de l'isopropanol et du butanol, et éventuellement de l'éthanol, ainsi que de l'acétone en phase aqueuse :
- (a) une section de séparation dudit mélange, par distillation comprenant au moins une colonne à bière (I) visant à éliminer une portion au moins de l'eau de la phase aqueuse pour obtenir un mélange concentré,
- (b) une section séparation du mélange concentré à l'étape a), par distillation comprenant au moins une colonne dite à acétone (II) visant à séparer l'acétone dudit mélange concentré pour obtenir un mélange concentré appauvri en acétone,
- (c) ladite section séparation par distillation comprenant au moins une colonne de distillation dite à isopropanol-butanol (III) et visant à séparer le mélange concentré appauvri en acétone obtenu à l'étape (b) en tête en un flux d'azéotrope eau-isopropanol ou eau-isopropanol-éthanol et en fond en un flux azéotrope eau-butanol,
- (d) ladite section de séparation par distillation visant à séparer le flux d'azéotrope eau-butanol obtenu à l'étape (c) en eau et en butanol, comprenant au moins un système de distillation hétéro-azéotropique comprenant au moins une colonne dite colonne à eau (IV) pour récupérer l'eau et au moins une colonne dite colonne à butanol (V) pour récupérer le butanol.

## Patentansprüche

1. Verfahren zur Extraktion von Alkoholen ausgehend von einem Ausgangsgemisch (1), das Alkohole, darunter mindestens Isopropanol und Butanol und optional Ethanol und/oder Aceton, in wässriger Phase umfasst, wobei das Verfahren eine Reihe von Trennvorgängen beinhaltet, darunter:
- eine Trennung durch Destillation, die durch mindestens eine sogenannte Isopropanol-Butanol-Destillationskolonne (III) durchgeführt wird, die am Kopf mit einem Verflüssiger (c3) ausgestattet ist, und darauf abzielt, das Ausgangsgemisch oder ein aus dem Ausgangsgemisch stammendes Gemisch am Kopf in einen Wasser-Isopropanol-Azeotrop-Strom (6) und am Sumpf in einen Wasser-Butanol-Azeotrop-Strom (7) zu trennen,
- eine Trennung durch Destillation, die darauf abzielt, den Wasser-Butanol-Azeotrop-Strom in Wasser und in Butanol zu trennen, und die durch ein Heteroazeotrop-Destillationssystem durchgeführt wird, das mindestens eine Kolonne, Wasserkolonne (IV) genannt, die das Wasser zurückgewinnt, und mindestens eine Kolonne, Butanolkolonne (V) genannt, die mit einem Verdampfer (r5) ausgestattet ist und das Butanol (9) zurückgewinnt, umfasst,
**dadurch gekennzeichnet, dass** man eine Wärmeübertragung von dem Wasser-Isopropanol-Azeotrop-Kopfstrom (6), der in den Verflüssiger der Isopropanol-Butanol-Destillationskolonne (III) eintritt, zu dem Strom, der in den Verdampfer der Butanolkolonne (V) eintritt, durchführt.

2. Verfahren nach dem vorhergehenden Anspruch, **dadurch gekennzeichnet, dass** man die Wärmeübertragung durch einen gemeinsamen Wärmetauscher (c3+r5) der beiden Kolonnen (III, V) durchführt, der den Verflüssiger (c3) und den Verdampfer (r5) beinhaltet.

3. Verfahren nach einem der vorhergehenden Ansprüche, **dadurch gekennzeichnet, dass** man die Funktionsweise der Isopropanol-Butanol-Destillationskolonne (III) regelt, um ein Flüssigkeit/Gas-Zweiphasengemisch in der Kolonne beizubehalten, indem insbesondere eine Temperatur von mindestens 120 °C und/oder ein Druck von mindestens 3 bar absolut vorgegeben wird.

4. Verfahren nach einem der vorhergehenden Ansprüche, **dadurch gekennzeichnet, dass** man den Druck der Isopropanol-Butanol-Destillationskolonne (III) auf einen Wert von mindestens 3 bar absolut, insbesondere mindestens 4 bar absolut, bevorzugt höchstens 10 bar absolut, insbesondere höchstens 7 bar absolut, insbesondere zwischen 4,5 und 6,5 bar absolut, regelt.

5. Verfahren nach einem der vorhergehenden Ansprüche, **dadurch gekennzeichnet, dass** man die Isopropanol-Butanol-Destillationskolonne (III) und die Butanolkolonne (IV) bei Temperaturen betreibt, die so gewählt sind, dass der Wasser-Isopropanol-Azeotrop-Kopfstrom (6) der Isopropanol-Butanol-Destillationskolonne (III) auf einer Temperatur T1 ist, die mindestens 8 °C, insbesondere mindestens 10 °C, über der Temperatur T2 des Stroms liegt, der den Sumpf der Butanolkolonne (V) verlässt.

6. Verfahren nach einem der vorhergehenden Ansprüche, **dadurch gekennzeichnet, dass** man die Isopropanol-Butanol-Destillationskolonne (III) und die Butanolkolonne (V) bei Temperaturen betreibt, die so gewählt sind, dass der Wasser-Isopropanol-Azeotrop-Kopfstrom (6) der Isopropanol-Butanol-Destillationskolonne (III) auf einer Temperatur T1 von mindestens 120 °C, insbesondere höchstens 140 °C, ist.

7. Verfahren nach einem der vorhergehenden Ansprüche, **dadurch gekennzeichnet, dass** das Ausgangsgemisch (1) auch Ethanol umfasst, und dadurch, dass die Trennung durch Destillation, die durch mindestens die Isopropanol-Butanol-Destillationskolonne (III) durchgeführt wird, darauf abzielt, das Gemisch oder ein aus dem Ausgangsgemisch stammendes Gemisch am Kopf in einen Wasser-Isopropanol-Ethanol-Azeotrop-Strom (6) und am Sumpf in einen Wasser-Butanol-Azeotrop-Strom (7) zu trennen.

8. Verfahren nach einem der vorhergehenden Ansprüche, **dadurch gekennzeichnet, dass** die Wärmeübertragung zu dem in die Butanolkolonne (V) eintretenden Strom die Gesamtheit der Wärme zuführt, die für den in den Verdampfer (r5) der Butanolkolonne (V) eintretenden Strom für den Betrieb der Kolonne erforderlich ist.

9. Verfahren nach einem der Ansprüche 1 bis 7, **dadurch gekennzeichnet, dass** die Wärmeübertragung zu dem in die Butanolkolonne (V) eintretenden Strom einen Anteil der Wärme zuführt, die für den in die Butanolkolonne (V) eintretenden Strom für den Betrieb der Kolonne erforderlich ist, wobei das Wärmekomplement von einer anderen Wärmequelle zugeführt wird, insbesondere von einer externen Wasserdampfquelle.

10. Verfahren nach einem der Ansprüche 1 bis 7, **dadurch gekennzeichnet, dass** die Wärmeübertragung zu dem in die Butanolkolonne (V) eintretenden Strom mehr Wärme zuführt, als für den in den Verdampfer (r5) der Butanolkolonne (V) eintretenden Strom für den Betrieb der Kolonne erforderlich ist, und dass man einen Kühler (c3') hinzufügt, um den Wärmeüberschuss abzuführen.

11. Verfahren nach einem der vorhergehenden Ansprüche, **dadurch gekennzeichnet, dass** es die folgende Reihe von Vorgängen zur Trennung des Ausgangsgemisches (1) umfasst, das Alkohole, darunter mindestens Isopropanol und Butanol und gegebenenfalls Ethanol sowie Aceton, in wässriger Phase umfasst:
- (a) eine Trennung des Gemisches durch Destillation, die in einer Bierkolonne (I) durchgeführt wird und darauf abzielt, mindestens einen Anteil des Wassers der wässrigen Phase zu entfernen, um ein konzentriertes Gemisch (2) zu erhalten,
- (b) eine Trennung des aus dem Schritt (a) hervorgegangenen konzentrierten Gemisches durch Destillation, die in einer sogenannten Acetonkolonne (II) durchgeführt wird und darauf abzielt, das Aceton des konzentrierten Gemisches (2) mindestens zum Teil zu trennen, um ein an Aceton verarmtes konzentriertes Gemisch (5) zu erhalten,
- (c) eine Trennung durch Destillation, die durch mindestens eine sogenannte Isopropanol-Butanol-Destillationskolonne (III) durchgeführt wird und darauf abzielt, das in dem Schritt (b) erhaltene an Aceton verarmte konzentrierte Gemisch am Kopf in einen Wasser-Isopropanol- oder Wasser-Isopropanol-Ethanol-Azeotrop-Strom (6) und am Sumpf in einen Wasser-Butanol-Azeotrop-Strom (7) zu trennen,
- (d) eine Trennung durch Destillation, die darauf abzielt, den in dem Schritt (c) erhaltenen Wasser-Butanol-Azeotrop-Strom in Wasser und in Butanol zu trennen, und die durch ein Heteroazeotrop-Destillationssystem durchgeführt wird, das mindestens eine Kolonne, Wasserkolonne (IV) genannt, die das Wasser zurückgewinnt, und mindestens eine Kolonne, Butanolkolonne (V) genannt, die das Butanol zurückgewinnt, umfasst.

12. Verfahren nach einem der vorhergehenden Ansprüche, **dadurch gekennzeichnet, dass** das Ausgangsgemisch (1), das Alkohole, darunter mindestens Isopropanol und Butanol und optional Ethanol und/oder Acton, in wässriger Phase umfasst, ein Most ist, der durch Vergärung von zuckerhaltigen Säften, die insbesondere aus lignocellulosehaltiger Biomasse stammen, unter der Einwirkung von Mikroorganismen erhalten wird, die insbesondere aus einem lösungsmittelproduzierenden Stamm hervorgegangen sind, bevorzugt unter mindestens einem der folgenden Mikroorganismen ausgewählt sind: Bakterien, insbesondere der Gattung *Clostridium, Escherichia coli,* Hefen, insbesondere vom Typ *Saccharomyces cerevisae.*

13. Verfahren nach einem der vorhergehenden Ansprüche, **dadurch gekennzeichnet, dass** das Ausgangsgemisch (1) mehrere Gemische mit Zusammensetzungen kombiniert, die unterschiedlich sind und jeweils einen oder mehrere Alkohole in wässriger Phase umfassen, insbesondere mehrere unterschiedliche Moste, die durch Vergärung von zuckerhaltigen Säften erhalten werden.

14. Anlage zur Extraktion von Alkoholen ausgehend von einem Ausgangsgemisch (1), das Alkohole, darunter mindestens Isopropanol und Butanol und gegebenenfalls Ethanol und/oder Aceton, in wässriger Phase umfasst, wobei die Anlage eine Reihe von Trennabschnitten beinhaltet, darunter:
- einen Abschnitt zur Trennung durch Destillation, die durch mindestens eine sogenannte Isopropanol-Butanol-Destillationskolonne (III) durchgeführt wird, die am Kopf mit einem Verflüssiger (c3) ausgestattet ist und darauf abzielt, das Ausgangsgemisch oder ein aus dem Ausgangsgemisch stammendes Gemisch am Kopf in einen Wasser-Isopropanol- oder Wasser-Isopropanol-Ethanol-Azeotrop-Strom (6) und am Sumpf in einen Wasser-Butanol-Azeotrop-Strom (7) zu trennen, - einen Abschnitt zur Trennung durch Destillation, die darauf abzielt, den Wasser-Butanol-Azeotrop-Strom (7) in Wasser und in Butanol zu trennen, und die durch ein Heteroazeotrop-Destillationssystem durchgeführt wird, das mindestens eine Kolonne, Wasserkolonne genannt, die das Wasser (8) zurückgewinnt, und mindestens eine Kolonne, Butanolkolonne genannt, die mit einem Verdampfer (r5) ausgestattet ist und das Butanol (9) zurückgewinnt, umfasst, **dadurch gekennzeichnet, dass** die Anlage einen Wärmetauscher umfasst, welcher der Isopropanol-Butanol-Destillationskolonne (III) und der Butanolkolonne (V) gemeinsam ist und eine Wärmeübertragung von dem Wasser-Isopropanol- oder Wasser-Isopropanol-Ethanol-Azeotrop-Kopfstrom (6) der Isopropanol-Butanol-Destillationskolonne (III), der in den Verflüssiger (c3) eintritt, zu dem Strom, der in den Verdampfer (r5) der Butanolkolonne (V) eintritt, gewährleistet, wobei der Wärmetauscher, der den beiden Isopropanol-Butanol- (III) und Butanol- (V) Destillationskolonnen gemeinsam ist, den Verflüssiger (c3) und den Verdampfer (r5) und optional einen zusätzlichen Verflüssiger (c3') am Kopf der Isopropanol-Butanol-Kolonne (III) beinhaltet, um den Wärmeüberschuss des Wasser-Isopropanol- oder Wasser-Isopropanol-Ethanol-Azeotrop-Kopfstroms (6) abzuführen.

15. Anlage nach Anspruch 14, **dadurch gekennzeichnet, dass** sie die folgende Reihe von Abschnitten zur Trennung des Ausgangsgemisches umfasst, das Alkohole, darunter mindestens Isopropanol und Butanol und gegebenenfalls Ethanol sowie Aceton, in wässriger Phase umfasst:
- (a) einen Abschnitt zur Trennung des Gemisches durch Destillation, umfassend mindestens eine Bierkolonne (I), die darauf abzielt, mindestens einen Anteil des Wassers der wässrigen Phase zu entfernen, um ein konzentriertes Gemisch zu erhalten,
- (b) einen Abschnitt zur Trennung des in dem Schritt a) konzentrierten Gemisches durch Destillation, umfassend mindestens eine sogenannte Acetonkolonne (II), die darauf abzielt, das Aceton des konzentrierten Gemisches zu trennen, um ein an Aceton verarmtes konzentriertes Gemisch zu erhalten,
- (c) wobei der Abschnitt zur Trennung durch Destillation mindestens eine sogenannte Isopropanol-Butanol-Destillationskolonne (III) umfasst und darauf abzielt, das in dem Schritt (b) erhaltene an Aceton verarmte konzentrierte Gemisch am Kopf in einen Wasser-Isopropanol- oder Wasser-Isopropanol-Ethanol-Azeotrop-Strom und am Sumpf in einen Wasser-Butanol-Azeotrop-Strom zu trennen,
- (d) wobei der Abschnitt zur Trennung durch Destillation, der darauf abzielt, den in dem Schritt (c) erhaltenen Wasser-Butanol-Azeotrop-Strom in Wasser und in Butanol zu trennen, mindestens ein Heteroazeotrop-Destillationssystem umfasst, das mindestens eine Kolonne, Wasserkolonne (IV) genannt, um das Wasser zurückzugewinnen, und mindestens eine Kolonne, Butanolkolonne (V) genannt, um das Butanol zurückzugewinnen, umfasst.

## Claims

1. Process for extracting alcohols from an initial mixture (1) comprising alcohols, including at least isopropanol and butanol, and optionally ethanol and/or acetone, in aqueous phase, said process comprising a series of separating operations, including:
- a distillative separation performed by at least one distillation column referred to as isopropanol-butanol column (III) and equipped at the top with a condenser (c3), and intended for separating said initial mixture or a mixture deriving from said initial mixture into a water-isopropanol azeotrope stream (6) at the top and into a water-butanol azeotrope stream (7) at the bottom,
- a distillative separation intended for separating the water-butanol azeotrope stream into water and butanol, performed by a hetero-azeotropic distillation system comprising at least one water-recovery column referred to as water column (IV), and at least one butanol (9)-recovery column referred to as butanol column (V) and equipped with a reboiler (r5),
**characterized in that** a heat transfer is performed from the top water-isopropanol azeotrope stream (6) entering the condenser of the isopropanol-butanol distillation column (III), to the stream entering the reboiler of the butanol column (V).

2. Process according to the preceding claim, **characterized in that** the heat transfer is performed by a heat exchanger (c3+r5) which is common to the two columns (III, V), incorporating said condenser (c3) and said reboiler (r5).

3. The process according to one of the preceding claims, **characterized in that** the operation of the isopropanol-butanol distillation column (III) is regulated for maintaining a two-phase liquid/gas mixture in the column, especially by imposing a temperature of at least 120°C and/or a pressure of at least 3 bar absolute.

4. Process according to one of the preceding claims, **characterized in that** the pressure of the isopropanol-butanol distillation column (III) is regulated to a value of at least 3 bar absolute, especially at least 4 bar absolute, preferably of at most 10 bar absolute, especially of at most 7 bar absolute, especially between 4.5 and 6.5 bar absolute.

5. Process according to one of the preceding claims, **characterized in that** the isopropanol-butanol distillation column (III) and the butanol column (IV) are operated at temperatures selected in such a way that the top water-isopropanol azeotrope stream (6) from the isopropanol-butanol distillation column (III) is at a temperature T1 at least 8°C, especially at least 10°C, greater than the temperature T2 of the stream exiting the bottom of the butanol column (V).

6. Process according to one of the preceding claims, **characterized in that** the isopropanol-butanol distillation column (III) and the butanol column (V) are operated at temperatures selected in such a way that the top water-isopropanol azeotrope stream (6) from the isopropanol-butanol distillation column (III) is at a temperature T1 of at least 120°C, especially of at most 140°C.

7. Process according to one of the preceding claims, **characterized in that** the initial mixture (1) also comprises ethanol, and **in that** the distillative separation which is performed by at least the isopropanol-butanol distillation column (III) is intended for separating said mixture or a mixture deriving from said initial mixture into a water-isopropanol-ethanol azeotrope stream (6) at the top and into a water-butanol azeotrope stream (7) at the bottom.

8. Process according to one of the preceding claims, **characterized in that** the thermal transfer to the stream entering the butanol column (V) provides the entirety of the heat required to the stream entering the reboiler (r5) of the butanol column (V) for the operation of said column.

9. Process according to one of Claims 1 to 7, **characterized in that** the thermal transfer to the stream entering the butanol column (V) provides a portion of the heat required to the stream entering the butanol column (V) for the operation of said column, the heat complement being supplied by another heat source, especially by an external steam source.

10. Process according to one of Claims 1 to 7, **characterized in that** the thermal transfer to the stream entering the butanol column (V) provides more heat than that required to the stream entering the reboiler (r5) of the butanol column (V) for the operation of said column, and **in that** a cooler (c3') is added for evacuating the excess heat.

11. Process according to one of the preceding claims, **characterized in that** it comprises the following series of operations for separating the initial mixture (1) comprising alcohols, including at least isopropanol and butanol, and optionally ethanol, and also acetone, in aqueous phase:
- (a) a separation of said mixture, by distillation performed in a beer column (I) and intended for removing at least a portion of the water from the aqueous phase, to give a concentrated mixture (2),
- (b) a separation of the concentrated mixture obtained from step (a), by distillation performed in a column referred to as acetone column (II), and intended for at least partly separating the acetone from said concentrated mixture (2), to give an acetone-depleted concentrated mixture (5),
- (c) the distillative separation performed by at least one distillation column referred to as isopropanol-butanol column (III) and intended for separating the acetone-depleted concentrated mixture obtained in step (b) into a water-isopropanol or water-isopropanol-ethanol azeotrope stream (6) at the top and into a water-butanol azeotrope stream (7) at the bottom,
- (d) the distillative separation intended for separating the water-butanol azeotrope stream obtained in step (c) into water and butanol, performed by a hetero-azeotropic distillation system comprising at least one water-recovery column referred to as water column (IV), and at least one butanol-recovery column referred to as butanol column (V).

12. Process according to one of the preceding claims, **characterized in that** the initial mixture (1) comprising alcohols, including at least isopropanol and butanol, and optionally ethanol and/or acetone, in aqueous phase is a must obtained by fermentation of sugary liquors, especially derived from lignocellulosic biomass, under the action of microorganisms, especially obtained from a solvent-producing strain, preferably selected from at least one of the following microorganisms: bacteria, especially of the genus *Clostridium, Escherichia coli,* yeasts, especially of *Saccharomyces cerevisiae* type.

13. Process according to one of the preceding claims, **characterized in that** the initial mixture (1) combines two or more mixtures of different compositions, each comprising one or more alcohols in aqueous phase, especially two or more different musts obtained by fermentation of sugary liquors.

14. Facility for extracting alcohols from an initial mixture (1) comprising alcohols, including at least isopropanol and butanol, and optionally ethanol and/or acetone, in aqueous phase, said facility comprising a series of separating sections, including:
- a section for distillative separation performed by at least one distillation column (III) referred to as isopropanol-butanol column and equipped at the top with a condenser (c3), and intended for separating said initial mixture or a mixture deriving from said initial mixture into a water-isopropanol or water-isopropanol-ethanol azeotrope stream (6) at the top and into a water-butanol azeotrope stream (7) at the bottom,
- a section for distillative separation intended for separating the water-butanol azeotrope stream (7) into water and butanol, performed by a hetero-azeotropic distillation system comprising at least one water (8)-recovery column referred to as water column, and at least one butanol (9)-recovery column referred to as butanol column and equipped with a reboiler (r5),
**characterized in that** the facility comprises a heat exchanger which is common to the isopropanol-butanol distillation column (III) and to the butanol column (V) and ensures a thermal transfer from the top water-isopropanol or water-isopropanol-ethanol azeotrope stream (6) from the isopropanol-butanol distillation column (III), entering the condenser (c3), to the stream entering the reboiler (r5) of the butanol column (V), said heat exchanger common to the two isopropanol-butanol distillation (III) and butanol (V) columns incorporating said condenser (c3) and said reboiler (r5), and optionally an additional condenser (c3') at the top of the isopropanol-butanol column (III) for evacuating the excess heat of the top water-isopropanol or water-isopropanol-ethanol azeotrope stream (6).

15. Facility according to Claim 14, **characterized in that** it comprises the following series of sections for separating the initial mixture comprising alcohols, including at least isopropanol and butanol, and optionally ethanol, and also acetone, in aqueous phase:
- (a) a section for separating said mixture, by distillation comprising at least one beer column (I) and intended for removing at least a portion of the water from the aqueous phase, to give a concentrated mixture,
- (b) a section for separating the concentrated mixture of step (a), by distillation comprising at least one column referred to as acetone column (II), and intended for separating the acetone from said concentrated mixture, to give an acetone-depleted concentrated mixture,
- (c) said section for distillative separation comprising at least one distillation column referred to as isopropanol-butanol column (III) and intended for separating the acetone-depleted concentrated mixture obtained in step (b) into a water-isopropanol or water-isopropanol-ethanol azeotrope stream at the top and into a water-butanol azeotrope stream at the bottom,
- (d) said section for distillative separation intended for separating the water-butanol azeotrope stream obtained in step (c) into water and butanol, comprising at least one hetero-azeotropic distillation system comprising at least one water-recovery column referred to as water column (IV), and at least one butanol-recovery column referred to as butanol column (V) .
